Europäisches Patentamt

European Patent Office

Office européen des brevets

(19)

(11) Publication number: 0 366 058
A2

(12) **EUROPEAN PATENT APPLICATION**

(21) Application number: 89119702.2

(51) Int. Cl.⁵: **A61K 9/16 , A61K 9/52**

(22) Date of filing: 24.10.89

(30) Priority: 27.10.88 US 263307

(43) Date of publication of application:
02.05.90 Bulletin 90/18

(84) Designated Contracting States:
AT BE CH DE ES FR GB IT LI NL

(71) Applicant: **ABBOTT LABORATORIES**
**One Abbott Park Road**
**Abbott Park, IL 60064-3500(US)**

(72) Inventor: **Thomas, Elizabeth**
**38660 Shagbark Lane**
**Wadsworth Illinois 60083(US)**
Inventor: **Butkus, Mary**
**2148 89th Street Apt. 1**
**Kenosha Wisconsin 53140(US)**
Inventor: **Allexon, Craig R.**
**3295 Susan Circle**
**Park City Illinois 60085(US)**
Inventor: **Mowles, Donald**
**901 E. Golf Road**
**Libertyville Illinois 60048(US)**
Inventor: **Riberal, Arthur R.**
**7650 Lowell Avenue**
**Skokie Illinois 60076(US)**
Inventor: **Swopes, Herman**
**2015 16th Street**
**North Chicago Illinois 60064(US)**

(74) Representative: **Modiano, Guido et al**
**MODIANO, JOSIF, PISANTY & STAUB**
**Modiano & Associati Via Meravigli, 16**
**I-20123 Milano(IT)**

(54) **Controlled-release delivery device, method for producing device, and method of using device.**

(57) An improved, controlled-release delivery device is comprised of a biologically active agent dispersed in a biodegradable lipid matrix. The agent-lipid matrix is in the form of microparticles. Because the lipid matrix exists as a solid at body temperature and because of its small size, the lipid delivery device has a high tolerance for the acid/enzyme environment of the GI tract. When administered by oral ingestion, the delivery device protects the GI tract from local irritation by the biologically active agent and promotes a high and uniform degree of bioavailability of the agent to the host.

**EP 0 366 058 A2**

FIG-1

DOG SERUM LEVELS OF TOTAL IRON
DOSE NORMALIZED FOR 105 Mg OF IRON        FASTED STATE
DOG STUDY-FER-DOG-01

LEGEND
□————□ MOL-IRON
○--------○ FeSO$_4$-MLD1
△—·—·—△ FeSO$_4$-MLD2

Y-axis: CONCENTRATION IN UG/DL

X-axis: HOURS AFTER DOSING

## CONTROLLED-RELEASE DELIVERY DEVICE, METHOD FOR PRODUCING DEVICE, AND METHOD OF USING DEVICE

### BACKGROUND OF THE INVENTION

#### 1. FIELD OF THE INVENTION

This invention relates to a biodegradable drug delivery device for administering biologically active agents to a mammalian host. In the oral administration of a biologically active agent destined for the blood stream, a degree of protection and specificity is desired. For example, controlled-release dosage forms which dissociate, in whole or in part, in the stomach can cause various ill effects, including irritation of the GI tract and/or undesirable tastes. Likewise, the premature dissolution of the biologically active agent in the stomach results in decreased and variable bioavailability of the agent to the bloodstream, and hence, decreased and variable therapeutic value of the controlled-release formulation. More particularly this invention relates to controlled-release delivery devices in the form of solid microlipid particles, to a method of forming the microlipid particles, and to a method of delivering biologically active agents to a mammalian host in a manner which protects the gastrointestinal tract from irritation and improves the bioavailability of the biologically active agent.

#### 2. DESCRIPTION OF RELATED ART

These problems are well known in the art and various compositions and constructions have been developed to overcome these disadvantages. For example, common delivery devices consist of granules or tablets containing biologically active agents which in turn are coated with a water-insoluble material such as a wax or synthetic resin. See, e.g., U.S. Pat. No. 3,062,720. However, these compositions are subject to enzymatic hydrolysis and are thus degraded, at least in part, within the gastrointestinal tract, resulting in irritation of the GI tract and lowered bioavailability of the agent to the bloodstream.

Compositions in which a biologically active agent is dispersed within a melt of water-insoluble material are described in U.S. Pat. No. 3,147,187. Likewise, controlled-delivery compositions are commercially available wherein a lipid material is added to particles of a biologically active agent, such as ferrous sulfate, as a hot melt coating. These controlled-release compositions are not entirely satisfactory due to the inherent instability of the resulting particles, their tendency to aggregate materially, and their relatively large size, all of which detract from their ability to serve as acceptable drug delivery devices.

Some of the more recent approaches disclosed for the controlled-delivery of biologically active agents include entrapping a macromolecular agent within copolymerized biodegradable microspheres (U.S. Pat. No. 4,741,872); compressing an admixture of biologically active agents and a binding agent into a tablet via a dry, direct compression process (U.S. Pat. No. 4,590,062); forming microreservoirs of phospholipid and phospholipid-immisible constituents (U.S. Patent No. 4,298,594); and encapsulating a pharmaceutical compound within a synthetic phosphatidyl compound having a modified polarhead moiety to increase the resistance of the phosphatidyl compound to phospholipase hydrylosis (U.S. Patent No. RE 31,609).

In spite of the numerous teachings and advances in the art, however, a continuing need arises for improved methods and greater efficiencies in the manufacture of controlled-release devices and the delivery thereof, particularly for controlled-release devices which are administered by oral ingestion.

### SUMMARY OF THE INVENTION

The present invention is directed to a biodegradable delivery device, a method for producing such device and a method for controllably delivering to a mammal a biologically active agent by means of such device. The present invention is based on the discovery that biologically active agents can be incorporated into a biodegradable lipid material to produce a biodegradable, controlled-release delivery device that is superior to conventional delivery devices. The lipid-agent dispersion is thereafter processed by art-recognized techniques into solid microparticles of a desired size, thereby entrapping and encapsulating the biologically active agents within the solid lipid particles.

Because the lipid materials exist as solids at body temperature and have a high tolerance for the

2

proteolytic enzymes and acid environment of the gastrointestinal tract, the microlipid particles exhibit excellent biological properties for oral ingestion. Because of the small size of the microlipid particles, they are not digested by the lipase enzymes of the lingual gland. Thus, the invention yields many advantages in that the use of such formulations protects the GI tract from local irritation which can be caused by the biologically active agent and further promotes a high and uniform degree of bioavailability of the agent to the host.

It is, therefore, an object of this invention to provide a biodegradable delivery device having a carrier matrix of the character described which is compatible with various biologically active agents and is non-toxic and biocompatible with the host system.

Another object of this invention is to provide a biodegradable delivery device which will resist degradation within the GI tract, thereby both minimizing irritation of the GI tract and enhancing the bioavailability of the agent for absorption into the bloodstream.

Yet another object of this invention is to provide an improved biodegradable drug delivery vehicle particularly suited for oral ingestion.

It is another primary object to provide an improved method for forming biodegradable controlled-release drug delivery vehicles.

Still a further object of this invention is the use of biodegradable microlipid delivery devices as means for controlled-release delivery of biologically active agents in warm-blooded vertebrates.

## BRIEF DESCRIPTION OF THE DRAWINGS

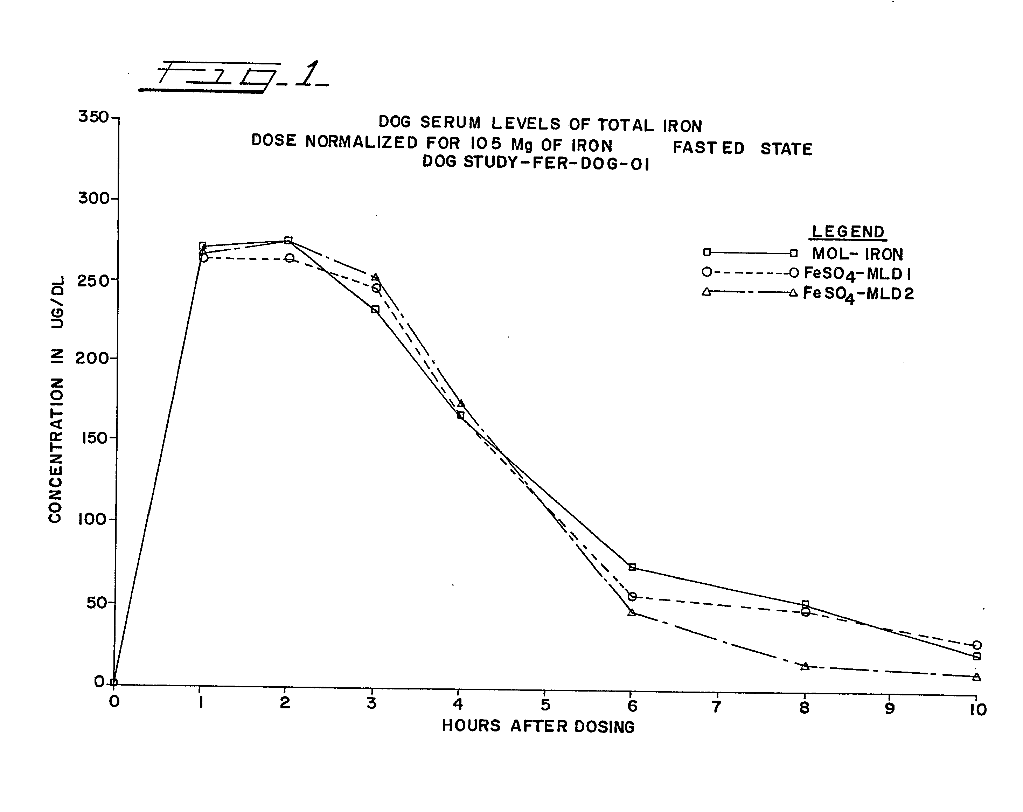

Fig. 1 illustrates normalized values of dog serum levels of total iron at various hourly intervals after oral dosing of a composition made in accordance with this invention.

## DETAILED DESCRIPTION OF THE INVENTION

The improved delivery devices of this invention comprise a biologically active agent dispersed in a solid biodegradable lipid particle. Due to the properties and structure of the microlipid delivery device, the captured biologically active agent assumes the properties of the lipid matrix and transverses the gastrointestinal tract without release. The microlipid particles are thereafter digested in the intestinal tract, whereby the biologically active agent is released in a manner which is highly efficient for absorption into the bloodstream.

The delivery devices can assume any one of a variety of alternative forms, including a powder, a compressed tablet, or a suspension of powder in a suitable liquid dosage form. The nature of the delivery device is not critical to the present invention, however, the improved delivery devices are particularly suited for the oral delivery of ferrous sulfate as an agent for the treatment of and/or prevention of iron deficiency in adults.

In general, the invention features a biodegradable lipid matrix in combination with a biologically active agent. The lipid materials useful as the delivery vehicle in this invention are non-toxic, biodegradable and bioresorbable, i.e., their degradation products are used by or are otherwise eliminated from the human body via existing biochemical pathways. The lipid constituent must be essentially insoluble in an aqueous environment and must remain solid at body temperature. Suitable lipid materials include natural and synthetic triglycerides and natural and synthetic sterols such as cholesterol and cholesterol esters. Preferred among the lipid materials are hydrogenated and partially hydrogenated long chain triglycerides. Especially preferred are long chain triglycerides having a high fraction of fatty acid chains with $C_{14}$ to $C_{18}$ carbon atoms. These triglycerides can be manufactured synthetically by known techniques or can be isolated from natural sources using known thermal or solvent fractionation methods.

As used herein, the term "biologically active agents" is intended to include vitamins, minerals, nutritional supplements, therapeutic drugs, proteins, hormones, peptides, vaccines, enzymes, enzyme inhibitors, and other pharmaceutical compositions. Various salts of the ferrous ion have been found to be especially suitable for controlled-release delivery by the method and device of the present invention. Preferred salts include sulfate, fumarate, succinate, glutamate, gluconate, and the like. Preferred among these ferrous salts is ferrous sulfate.

In addition to the foregoing materials, it may be desirable to incorporate other components such as binding agents, release agents, or absorption enhancers in a manner well known in the art.

The preparation of the present microlipid delivery device typically involves combining the biologically active agent with a lipid carrier. Preferably, the weight percent of biologically active agent present in the lipid ranges from about 5 percent to about 50 percent and the weight percent of the lipid material ranges from about 50 percent to about 95 Percent. The lipid carrier is heated until melted to form a liquid, prior to mixing with the biologically active agent. In a preferred embodiment of this invention, the lipid phase of the delivery formulation is about 80 to about 90 percent by weight and the biologically active agent is present in an amount ranging from about 10 to about 20 percent by weight of the total formulation weight. The ratio of lipid phase to biologically active agent phase can be adjusted to provide the functional characteristics warranted by any given application of the controlled-release device.

The lipid-agent mixture is homogenized, if necessary, to reduce the size of the suspended agent particles. The mixture is then solidified to form microlipid particles of a desired size by spray congealing or by some other art-recognized technique. The preferred maximum diameter of the microlipid particles is about 500 microns. Especially preferred are microlipid particles having particle diameters of about 50 microns to about 250 microns.

The release rate from a delivery system based on the present compositions is a function of the properties of the solid lipid matrix, the size of the liquid particles, and the "in vivo environment" of the ingested delivery device. The non-porous lipid matrix is solid at body temperature and stable in the acid/enzyme environment of the gastric fluid of the stomach. The microlipid particle is not digested by acid lipases secreted by the lingual gland because of its small size. The microlipid delivery device creates a hydrophobic domain within which the drug substance is carried, thereby preventing the release of the drug while transversing the gastrointestinal tract. The lipid matrix is thereafter digested by lipolytic enzymes present in the microvillus membrane of the duodenum releasing the biologically active agent in the manner which is highly efficient for absorption into the bloodstream.

In a preferred embodiment of this invention, a partially hydrogenated long chain triglyceride is used to form the lipid carrier phase. Ferrous sulfate salts are dispersed in the melted triglyceride in ratios ranging from about 5 percent to about 50 percent by weight. The liquid, lipid-ferrous sulfate combination is then spray congealed to form finely divided particles having maximum particle diameters of about 500 microns.

The use of the present compositions as drug delivery devices allows for a significant degree of control over the release of drugs administered orally and otherwise. Such control not only provides a high and uniform rate of bioavailability of the drug but also serves the important function of protecting the GI tract from unpleasant, irritating properties associated with various biologically active agents.

The controlled-release delivery system in accordance with the present invention is especially suited for administration by oral ingestion, however, alternative methods of administration such as implantation also are contemplated. It further will be appreciated by those skilled in the art that the microlipid particles of the present invention may be administered alone as a powder, as a dry, compressed tablet, or can be processed into a liquid suspension suitably selected with respect to oral administration and conventional pharmaceutical practices.

The present invention is further illustrated by the following examples, none of which are to be construed as limiting the invention in any respect.

## EXAMPLE 1

45 Kg of partially hydrogenated fat (Durkee 07) was added to a stainless steel vat. The fat was heated until melted by a steam jacket around the vat and heating the vat to a temperature of 75-85° C. 5 Kg of $FeSO_4$ was added and mixed with the liquid fat to form a uniform dispersion of $FeSO_4$ in the fat.

The $FeSO_4$/fat mixture was transferred to a spray congeal tower and pumped under pressure (1000-1500 psi) out of the nozzle as a fine mist. The material congealed into a fine, free-flowing powder. The powder was passed through a sieve having a 250 micron screen. The $FeSO_4$ content of the resulting $FeSO_4$/fat composition was assayed via atomic absorption and determined to be 9.0 percent by weight of the total composition. This compared to a theoretical value of 10 percent.

## EXAMPLE 2

10 Kg of $FeSO_4$ was mixed with 40 Kg of partially hydrogenated fat (Durkee 07) according to the procedure of Example 1. The $FeSO_4$ content of the resulting $FeSO_4$/fat composition was assayed via atomic absorption and determined to be 19.5 percent by weight of the total composition. This compared to a theoretical value of 20 percent.

<u>EXAMPLE 3</u>

In vivo tests of biodegradable controlled-release delivery devices prepared according to the method of Examples 1 and 2 were conducted on anemic dogs. The study was designed to assess both the bioavailability and GI irritation associated with a microlipid preparation of $FeSO_4$, designated "$FeSO_4$-MLD" according to the present invention, as compared to the commercial $FeSO_4$ dosage form MOL-IRON$_R$ ferrous sulfate tablets (Schering Cor,.).

Ferrous sulfate - MLD (microlipid delivery) formulations were suspended in a mixture of 70% sucrose and 0.1% Xanthan gum to yield 105 mg of elemental iron in a 10 ml aliquot. The dosing solutions were prepared as follows..

Table I

| Ferrous Sulfate - MLD Formulations | | | | |
|---|---|---|---|---|
| Formulation No. | $FeSo_4$ | Fe-mg per gram | 105 mg Fe | Dosing Solution |
| | (By wt %) | | (In Bulk) | (in 50 ml) |
| $FeSo_4$-MLD 1 | 7.1%(9.0%) | 26.1mg | 4.02g | 21.0g |
| $FeSO_4$-MLD 2 | 15.4%(19.5%) | 56.6mg | 1.85g | 9.25g |

The MOL-IRON$_R$ ferrous sulfate tablet used in this study was not a controlled-release formulation. Each MOL-IRON$_R$ tablet contained 195 mg ferrous sulfate (39 mg elemental iron). Thus, each dog was orally dosed with three MOL-IRON$_R$ tablets (or 117 mg elemental iron) to closely approximate the dosing at 105 mg of iron per day.

Twelve beagle dogs were parental oral-dosed with the equivalent of 105 mg of elemental iron from each ferrous sulfate preparation in a crossover design. Serum samples were collected for ten hours post dosing and analyzed for iron by Ferozine methods (Stookey, <u>Anal. Chem.</u> 42:779, 1970; Persijn et al., <u>Clin. Chem. Acta.</u> 35:91, 1971). Serum levels of iron exceeding baseline values and subsequent area under the curve (AUC) values were calculated as follows:

Table II

| | AUC | Cmax | Tmax |
|---|---|---|---|
| | ug/hr/mL | ug/mL | Hr. |
| $FeSo_4$-MLD 1 | 1674 | 361 | 1.33 |
| $FeSO_4$-MLD 2 | 1511 | 356 | 1.44 |
| Mol-Iron$_R$ | 1451 | 327 | 1.33 |

Fig. 1 is a plot of time versus serum $FeSO_4$ concentrations for each of the $FeSO_4$ preparations listed above in Tables I and II. Fig. 1 reports the extent and rate of absorption of iron in dog serum samples for each of the $FeSO_4$ preparations, with dosages normalized to 105 mg of $FeSO_4$.

GI irritation values were determined for each dog group and are shown in Table II:

Table III

|  | Irritation per Group |
|---|---|
| $FeSO_4$-MLD 1 | 33% |
| $FeSO_4$-MLD 2 | 25% |
| Mol-Iron$_R$ | 50% |

These results demonstrate that, as compared to a conventional $FeSO_4$ commercial product, the $FeSO_4$-MLD formulations reduced GI irritation.

Table IV further summarizes the decreased GI irritation, as manifested by vomitting and diarrhea, realized in dogs which had been administered iron preparations prepared in accordance with this invention.

## Table IV

### Summary of Adverse Effects*

| FeSO$_4$ Formulation | Dog No. | Study No. | Hours After Dosing | | | | |
|---|---|---|---|---|---|---|---|
| | | | 0-1 HR | 1-2 HR | 2-3 HR | 3-4 HR | 4-8 HR |
| MOL-IRON | | | | | | | |
| | 9 | 1 | E-H | | | | |
| | 11 | 1 | | E-L | | | |
| | 13 | 2 | | E-M | | | |
| | 14 | 2 | E-H | | | | |
| | 16 | 3 | E-M | E-M | | | |
| | 17 | 3 | E-M | | | | D |
| FeSO$_4$-MLD-1 | | | | | | | |
| | 10 | 2 | | | | | D |
| | 11 | 2 | E-H | | | | |
| | 14 | 1 | | E-M | | | |
| | 20 | 3 | E-M | | | | |
| FeSO$_4$-MLD-2 | | | | | | | |
| | 13 | 3 | E-H | | | | |
| | 14 | 3 | E-M | | | | |
| | 20 | 2 | | | | E-M | |

*D = Diarrhea  
 E = Emesis  
 L = Light  
 M = Medium  
 H = Heavy

This table shows that dogs which had received oral iron dosages in the form of FeSO$_4$-MLD preparations demonstrated greater tolerance for such oral iron dosages, as compared to MOL-IRON$_R$ ferrous sulfate preparations.

These results illustrate that the FeSO$_4$-MLD formulations of the present invention provide equivalent or enhanced bioavailability of total iron to the host, while at the same time reduce the GI irritation associated with the oral administration of FeSO$_4$.

While the present invention has been described herein with some particularity, those of skill in the art will recognize numerous modifications and variations which remain within the spirit of the invention. These modifications and variations are within the scope of the invention as described and claimed herein.

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

**Claims**

1. A biodegradable drug delivery device adapted for the controlled release of a biologically active agent within a mammalian host, comprising a lipid matrix which is solid at body temperature and a biologically active agent in said lipid matrix, said delivery device being in the form of microparticles having maximum diameters of about 500 microns.

2. The delivery device of Claim 1 wherein the lipid matrix is selected from the group consisting of sterol and triglycerides and combinations thereof.

3. The delivery device of Claim 2 wherein the triglyceride is selected from the group consisting of partially hydrogenated long chain triglycerides and hydrogenated long chain triglycerides and combinations thereof.

4. The delivery device of Claim 1 wherein the biologically active agent is selected from the group consisting of vitamins, minerals, nutritional supplements, proteins, hormones, peptides, vaccines, enzymes and pharmaceutical compositions.

5. The delivery device of Claim 1 wherein the biologically active agent is a ferrous salt selected from the group consisting of ferrous sulfate, ferrous fumarate, ferrous succinate, ferrous glutamate, ferrous gluconate and ferrous lactate.

6. A method for forming a biodegradable microlipid delivery device, comprising:
melting a lipid material which exists as a solid at body temperature and which is capable of forming a non-toxic, biocompatible, water-insoluble carrier fluid;
adding a biologically active agent to said carrier fluid to form a lipid-agent dispersion;
solidifying said lipid-agent dispersion to form particles consisting of said biologically active agent embedded in a solid lipid carrier matrix, said lipid-agent particles having a maximum diameter of about 500 microns.

7. The method of Claim 6 wherein the weight percent of biologically active agent present in the lipid-agent dispersion ranges from about 5 percent to about 50 percent and the weight percent of lipid material ranges from about 50 percent to about 95 percent.

8. The method of Claim 6 wherein the step of solidifying the lipid-agent dispersion to form particles comprises spray congealing said dispersion.

9. The biodegradable microlipid delivery device produced in accordance with the method of Claim 6.

10. A method of controllably delivering to a mammalian host a biologically active agent, comprising introduced into said host a biologically active agent contained within microparticles said microparticles being in solid form at body temperature, having maximum diameters of about 500 microns, and being formed of a lipid material, thereby imparting to said microparticles a structure in which contact between said biologically active agent and the gastrointestinal tract of said mammalian host is minimized, and thereby protecting the gastrointestinal tract from irritation and providing the controlled release of said biologically active agent therefrom in a manner which improves the bioavailability of said agent.

FIG-1

DOG SERUM LEVELS OF TOTAL IRON
DOSE NORMALIZED FOR 105 Mg OF IRON          FASTED STATE
DOG STUDY-FER-DOG-01

LEGEND
□————□ MOL- IRON
O------O FeSO₄-MLD I
△—·—△ FeSO₄-MLD 2

CONCENTRATION IN UG/DL

HOURS AFTER DOSING

EP 0 366 058 A2